# EUROPEAN PATENT SPECIFICATION

(11) **EP 1 280 562 B1**
(45) Date of publication and mention of the grant of the patent: **01.12.2004**
(21) Application number: 01940405.2
(22) Date of filing: 03.05.2001
(51) Int. Cl.: A61L 27/20, A61L 27/38

(54) **BIOMATERIALS COMPRISED OF PREADIPOCYTE CELLS FOR SOFT TISSUE REPAIR**
PREADIPOZYTEN ENTHALTENDE BIOMATERIALIEN ZUR REGENERATION VON WEICHGEWEBE
BIOMATERIAUX COMPOSES DE CELLULES PREADIPOCYTES CON US POUR REPARER DES TISSUS MOUS

(30) Priority: 03.05.2000 US 201984 P
(43) Date of publication of application: 05.02.2003
(73) Proprietor: FIDIA ADVANCED BIOPOLYMERS S.R.L., 35031 Abano Terme (Padova) (IT); VON HEIMBURG, Dennis, 52072 Aachen (DE)
(72) Inventor: VON HEIMBURG, Dennis, 52072 Aachen (DE); PAVESIO, Alessandra, I-35141 Padova (IT)
(74) Representative: Plougmann & Vingtoft a/s
(86) International application number: PCT/EP2001/005087
(87) International publication number: WO 2001/082991

(56) References cited:
- WO-A-00/01733
- WO-A-00/37124
- WO-A-97/18842
- WO-A-98/56897
- WO-A-99/24070
- WO-A-99/65534

## Description

### SUMMARY OF THE INVENTION

The present invention is directed to a biomaterial for soft tissue reconstruction comprised of a support material comprised of a benzyl ester of hyaluronic acid and preadipocyte cells seeded on said support material, to an injectable preparation for filling soft tissue defects and depressions comprised of a totally water soluble hyaluronic acid derivative or a partially water soluble hyaluronic acid derivative and preadipocyte cells suspended in the preparation, and to the use of these support materials and injectable preparations.

### BACKGROUND OF THE INVENTION

The correction of soft tissue defects is an important challenge in plastic and reconstructive surgery. Adipose tissue as a free graft has been used for the reconstruction of soft tissue defects for more than 100 years. But there has been a lack of an optimal implant material for soft tissue replacement. Free adipose tissue grafts are used but the results are poor and unpredictable. The transplants are largely absorbed and replaced by fibrous tissue and oil cysts. The recently revived technique of injecting aspirated fat fragments also gives unsatisfactory results, ranging from 50% shrinkage of the graft to complete resorption. The poor results of free fat autotransplantation are thought to be due to the low tolerance of the fat cells to ischemia and the slow rate of revascularisation.

Adipose precursor cells located in the stroma of adipose tissue can be isolated and cultured. These cells demonstrate *in vitro* differentiation and dedifferentiation under different conditions and are a possible source for soft tissue engineering because of the ability to proliferate and differentiate. In a preliminary study we observed that preadipocytes revascularise rapidly and reaccumulate fat after transplantation. In a recent study rat preadipocytes differentiated in PLGA scaffolds after grafting.

Recently, mesenchymal stem cells (MSCs), obtained from adult bone marrow, have been used for the production of various tissue cell types. These isolated stem cells are not totipotent, as are embryonic stem cells, but pluripotent and capable of differentiating into connective tissue and its derivatives. Mesenchyme is a source not only of connective tissue such as muscle, tendon and ligament, but also blood, cartilage, bone, fat cells and the outer layers of blood vessels. To date, MSCs have been successfully differentiated into adipose cells, chrondrocyte cells and osteocyte cells (Pittenger et al. (1999) Science 284:143-7).

But there is still a need for a good bioartificial soft tissue filler for tissue engineering, one that ideally would be a delivery vehicle to support human preadipocytes in grafting procedures. The material should provide a structure for supporting implanted cells (pre-adipose cells) and a structure that allows the cells to invade and differentiate after transplantation. Introduction of endothelial cells, which are angiogenic, would also enhance the performance of bioartificial soft tissue filler material. A higher number of mature adipocytes are found in well vascularized adipose tissue, possibly due to the influence that endothelial cells have on differentiating preadipocytes and adipocytes. Consequently, endothelial cells are particularly useful in adipose tissue engineering that is based on preadipocytes. Mechanical stability of the carrier is also important and the material/carrier may not be resorbed too quickly after transplantation.

These needs are met by the present invention which provides an optimal matrix for isolated and cultured human preadipocytes, mesenchymal stem cells and endothelial cells *in vitro* and *in vivo*. The present invention is also useful as a bioartificial soft tissue filler material, particularly as a scaffold for preadipocytes, mesenchymal stem cells and/or endothelial cells with the ability to support *in vivo* adipogenesis.

### BRIEF DESCRIPTION OF THE FIGURES

Fig. 1 Human preadipocytes 24 hours after being seeded on HYAFF 11 sponge (HS+). Good adherence of viable cells to the scaffold can be observed. Cytoplasmic vacuoles are seen and these store lipid as typical morphological signs of differentiation. (toluidine-blue, sc=scaffold).
Fig. 2 Macroscopic appearance of explanted HS grafts after 3 weeks in the nude mouse. A thin yellow tissue presented on the preadipocyte grafts with new vessel formation (right). The contralateral control sponge out of the same animal revealed almost no change to the sponge and no vessels (left).
Fig. 3 Microscopical view of the HS+ section after 3 weeks in the nude mouse. Differentiated clusters of adipocytes are more numerous in open pores even in the centre of the sponge. Note the intense red stain of lipid containing mature adipocytes and the scaffold structure. (oil-red, sc=scaffold)
Fig. 4 Invaded human cells in preadipocyte/scaffold grafts HS+ in group A (3 weeks in vivo). Note good and homogenous distribution of human cells in the scaffold. (mah-vim stain, sc=scaffold)
Fig. 5 Cellularity of donor and host cells in preadipocyte/scaffold constructs and controls.
Fig. 6 Ultrastructure of preadipocytes in the nonwoven matrix after 3 weeks in vivo. The cells contain multiple cytoplasmic lipid droplets. The fibers and preadipocytes are closely packed together. Note the HYAFF®11 fiber in the left above comer and the new ECM inbetween.
Fig. 7 Differentiated adipocytes in a cluster in HYAFF®11 sponge after 8 weeks in vivo. The cells contain single lipid droplets of large sizes (>50µm) and show typical signet ring appearance. Note the new collagen fibers in between the cells.

### DETAILED DESCRIPTION OF THE INVENTION

Soft tissue defect correction by plastic or reconstructive surgery can be performed by implantation of isolated and culture-expanded adipose precursor cells, MSCs and/or endothelial cells. Adipose precursor cells, when implanted, differentiate into adipocytes, which are animal connective tissue cells that are specialized for the synthesis and storage of fat. Although MSCs may, in some cases, first require in vitro manipulation to initiate differentiation, these cells are also capable of producing adipocytes. But appropriate supports or scaffolds are needed in this soft tissue engineering to allow and encourage differentiation and proliferation of the precursor cells, MSCs or endothelial cells.

In the present invention, the biomaterial for soft tissue reconstruction is comprised of (a) a support material comprised of a benzyl ester of hyaluronic acid and (b) preadipocyte cells seeded on said support material. The support material may be in the form of a sponge or non-woven material. Alternatively, the biomaterial is an injectable preparation for filling soft tissue defects and depressions comprised of (a) a totally water soluble hyaluronic acid derivative or a partially water soluble hyaluronic acid derivative and (b) preadipocyte cells suspended in the preparation. The derivatives are particularly a benzyl ester or an amide derivative. In particular, benzyl ester derivatives with 85% or less esterification and the dodecylamide of HA are preferred for injectable preparations. The preparation of such benzyl esters is described in EP 0 216 453 B1 and the preparation of amide derivatives is described in WO 00/01733.

The term "hyaluronic acid" (also referred to as "HA" hereinafter) is used in literature to designate an acidic polysaccharide with various molecular weights constituted by residues of D-glucoronic acid and N-acetyl-D-glucosamine, which naturally occur in cellular surfaces, in the basic extracellular substances of the connective tissue of vertebrates, in the synovial fluid of joints, in the vitreous humor of the eye, in the tissue of the human umbilical cord and in cocks' combs.

Hyaluronic acid plays an important role in an organism, firstly as mechanical support of the cells of many tissues, such as the skin, the tendons, muscles and cartilage and it is, therefore, the main component of the intracellular matrix. But hyaluronic acid also performs other functions in the biological processes, such as the hydration of tissues, lubrication, cellular migration, cell function and differentiation. (See for example A. Balazs et al., Cosmetics & Toiletries, No. 5/84, pages 8-17). Hyaluronic acid may be extracted from the above mentioned natural tissues, such as cocks' combs, or also from certain bacteria. Today, hyaluronic acid may also be prepared by microbiological methods. The molecular weight of whole hyaluronic acid obtained by extraction is in the region of 8-13 million. However, the molecular chain of the polysaccharide can be degraded quite easily under the influence of various physical and chemical factors, such as mechanical influences or under the influence of radiation, hydrolyzing, oxidizing or enzymatic agents. For this reason often in the ordinary purification procedures or original extracts, degraded fractions with a lower molecular weight are obtained. (See Balazs et al. cited above). Hyaluronic acid, its molecular fractions and the respective salts have been used as medicaments and their use is also proposed in cosmetics (see for example the above mentioned article by Balazs et al. and French Patent No. 2478468).

As a therapeutic agent, hyaluronic acid and its salts have been used especially in therapy for arthropathies, such as in veterinary medicine for the cure of arthritis in horses [Acta Vet. Scand. 167, 379 (1976)]. As an auxiliary and substitutional therapeutic agent for natural tissues and organs, hyaluronic acid and its molecular fractions and their salts have been used in ophthalmic surgery (see for example Balazs et al., Modern Problems in Ophthalmology, Vol. 10, 1970, p. 3-E.B. Strieff, S. Karger eds., Basel; Viscosurgery and the Use of Sodium Hyaluronate During Intraocular Lens Implantation, Paper presented at the International Congress and First Film Festival on intaocular Implantation, Cannes, 1979; U.S. Patent No. 4,328,803 with a summary of the literature on the uses of HY in ophthalmology; and U.S. Patent No. 4,141,973.) European patent publication no. 0138572 describes a molecular fraction of hyaluronic acid which can be used, for example, as sodium salt for intraocular and intraarticular injections suitable for the substitution of internal fluids of the Eye and in arthropathy therapies.

Hyaluronic acid may also be used as an additive for a wide variety of polymeric materials used for medical and surgical articles, such as polyurethanes, polyesters, polyolefins, polyamides, polysiloxanes, vinylic and acrylic polymers and carbon fibers with the effect of rendering these materials biocompatible. In this case the addition of HY or one of its salts is effected for example by covering the surface of such materials, by dispersion in the same or by both of these procedures. Such materials may be used for the manufacture of various sanitary and medical articles, such as cardiac valves, intraocular lenses, vascular clips, pacemakers and such (see U.S. Patent No. 4,500,676).

Although the term "hyaluronic acid" is commonly used in an improper sense, meaning, as can be seen from above, a whole series of polysaccharides with alternations of residues of D-gtucuronic acid and N-acetyl-D-glucosamine with varying molecular weights or even degraded fractions of the same, and although the plural form "hyaluronic acids" may seem more appropriate, the discussion herein shall continue to use the singular form to refer to hyaluronic acid in its various forms including its molecular fractions, and the abbreviation "HA" will also often be used to describe this collective term.

EP 0 216 453 B1 describes total or partial esters of hyaluronic acid with an alcohol of the aliphatic, or araliphatic series or a salt of such partial ester with an inorganic or organic base. Such esters possess interesting bio-plastic and pharmaceutical properties and may be used in various fields, including cosmetics, surgery and medicine. In the case of hyaluronic acid, in which the new products qualitatively possess the same or similar physical-chemical, pharmacological and therapeutic properties, they are considerably more stable, especially regarding the action of the natural enzymes responsible for the degradation of the polysaccharaide molecule in the organism, such as especially hyaluronidase, and they, therefore, conserve the above mentioned properties for very long periods.

WO 00/01733 describes amides of hyaluronic acid and derivatives thereof obtained by reacting the carboxy groups or amino groups originating from deacetylation reactions with amines, and acids of the aliphatic, aromatic, arylaliphatic, cycloaliphatic, heterocyclic series, and without the use of spacer chains. These compounds can be either water soluble or insoluble, according to the acid, the amine, the percentage of amide bond or the derivative of hyaluronic acid used to prepare the amide. These amides can be used in various fields of surgery, in the prevention of post-surgical adhesions and hypertrophic scarring, cardiology, dermatology, opthalmology, otorhinolaryngology, dentistry, orthopaedics, gyaecology, urology, extra-corporeal blood circulation and oxygenation, cosmetics and angiology. Like the esters described above, these amides of hyaluronic acid retain the viscosity of free hyaluronic acid, but are more stable and persist longer before being degraded.

WO 99/24070 describes a biomaterial comprising a benzylester derivative of hyaluronic acid in association with cells such as stem cells or adipocytes. This biomaterial is intended for use in soft tissue reconstruction.

WO 97/18842 describes compositions comprising a benzyl ester derivative of hyaluronic acid is association with bone marrow mesenchymal stem cells for use in soft tissue reconstruction such as skin reconstruction.

WO 98/56897 describes hyaluronic acid derivatives compositions comprising endothelial cells for skin transplants.

In the present invention, the ester of HA with benzyl alcohol (the benzyl ester) or an amide of HA is utilized in the support or scaffold for adipose precursor cells. The benzyl ester of HA utilized in the invention is preferably either a "total ester" (that is, a derivative wherein all of the carboxyl groups of the HA are esterified with benzyl alcohol) or a 5-99% ester (that is, a derivative wherein 5 to 99% of the carboxyl groups are esterified and the remaining groups salified). These derivatives provide preferred scaffold support materials for cultivating and growth of human preadipocytes, MSCs and/or endothelial cells. These derivatives can also be delivered with accompanying populations of cells (preadipocytes, mesenchymal stem cells or endothelial cells) by injection. Here, for example, a benzyl ester or an amide of HA is mixed with a population of preadipocytes and/or MSCs and/or endothelial cells and then injected into a site of soft tissue containing a depression, defect, wrinkle or deformity. Hyaluronic acid derivatives, especially those wherein 85% or less of the carboxyl groups of the HA are esterified with benzyl alcohol and the dodecyl amide of HA, are particularly preferred. One especially preferred combination is the dodecyl amide of HA and preadipocyte cells.

The benzyl esters, as noted above, can be prepared according to the procedures described in EP 0 216 453 B1 (see Examples 1-4). The amides can be prepared according to the procedures described in WO 00/01733 (see Examples 5-24.

The following examples 1-24 are preparative examples teaching how to synthesise hyaluronic acid derivatives used in the invention.

### Example 1 - Preparation of the benzylester of hyaluronic acid (HY)

12.4 g of HY tetrabutylammonium salt with a molecular weight of 170,000 corresponding to 20 m.Eq. of a monomeric unit are solubilized in 620 ml of dimethylsulfoxide at 25°, 4.5 g (25 m. Eq.) of benzyl bromide and 0.2 g of tetrabutylammonium iodide are added, the solution is kept for 12 hours at 30°.

The resulting mixture is slowly poured into 3,500 ml of ethyl acetate under constant agitation. A precipitate is formed which is filtered and washed four times with 500 ml of ethyl acetate and finally vacuum dried for twenty four hours at 30°.

9 g of the benzyl ester product in the title are obtained. Quantitative determination of the ester groups is carried out according to the method described on pages 169-172 of Siggia S. and Hanna J.G. "Quantitative organic analysis via functional groups" 4^{th} edition, John Wiley and Sons.

### Example 2 - Preparation of the benzyl ester of hyaluronic acid HY

3 g of the potassium salt of HY with a molecular weight of 162,000 are suspended in 200 ml of dimethylsulfoxide; 120 mg of tetrabutylammonium iodide and 2.4 g of benzyl bromide are added.

The suspension is kept in agitation for 48 hours at 30°. The resulting mixture is slowly poured into 1,000 ml of ethyl acetate under constant agitation. A precipitate is formed which is filtered and washed four times with 150 ml of ethyl acetate and finally vacuum dried for twenty four hours at 30°.

3.1 g of the benzyl ester product in the title are obtained. Quantitative determination of the ester groups is carried out according to the method described on pages 169-172 of Siggia S. and Hanna J.G. "Quantitative organic analysis via functional groups" 4^{th} edition, John Wiley and Sons.

The scaffold support material is comprised of a spongy material comprised of the HA benzyl ester, and can be prepared as follows.

### Example 3 - Preparation of a spongy material made with hyaluronic acid esters

1 g of benzyl esters of hyaluronic acid with a molecular weight of 170,000 in which all the carboxylic groups are esterified (obtained for example as described above) are dissolved in 5 ml of dimehtylsulfoxide. To each 10 ml of solution prepared, a mixture of 31.5 g of sodium chloride with a degree of granularity corresponding to 300µ, 1.28 g of sodium bicarbonate and 1 g of citric acid is added and the whole is homogenized in a mixer.

The pasty mixture is stratified in various ways, for instance by means of a mange consisting of two rollers which turn opposite each other at an adjustable distance between the two. Regulating this distance the past is passed between the rollers together with a strip of silicone paper which acts as a support to the layer of paste thus formed. The layer is cut to the desired dimensions of length and breadth, removed from the silicone, wrapped in filter paper and emerged in a suitable solvent, such as water. The sponges thus obtained are washed with a suitable solvent, such as water, and optionally sterilized with gamma rays.

A scaffold support comprised of a non-woven material of the HA benzyl ester (also known as HYAFF®11) can be prepared as described in U.S. Patent 5,520,916 according to the following procedures.

### Example 4

A solution of HYAFF®11 in dimethylsulfoxide at a concentration of 135 mg/ml is prepared in a tank and fed by a gear metering pump into a spinneret for wet extrusion composed of 3000 holes each measuring 65 microns.

The extruded mass of threads passes into a coagulation bath containing absolute ethanol. It is then moved over transporting rollers into two successive rinsing baths containing absolute ethanol. The drafting ratio of the first roller is set at zero while the drafting ratio between the other rollers is set at 1.05. Once it has been passed through the rinsing baths, the hank of threads is blown dry with hot air at 45.degree.-50.degree. C. and cut with a roller cutter into 40 mm fibers.

The mass of fibers thus obtained is tipped into a chute leading to a carding/cross lapping machine from which it emerges as a web, 1 mm thick and weighing 40 mg/mq. The web is then sprayed with a solution of HYAFF®11 in dimethylsulfoxide at 80 mg/ml, placed in an ethanol coagulation bath, in a rinsing chamber, and lastly in a drying chamber.

The final thickness of the material is 0.5 mm.

### Example 5

### Preparation of partially N-deacetylated hyaluronic acid in the form of sodium salt (DHA/Na)

One gram of sodium hyaluronate, with a mean molecular weight of 600 Kda, is solubilized in 50 ml of a 1% solution of hydrazine sulphate in hydrazine monohydrate. This is left to react under agitation for five days (120 hours) at 55°C, after which the reaction is slopped by adding 100 ml of ethanol. The precipitate thus formed Is filtered through a Gooch crucible, washed with ethanol and then dried at room temperature at reduced pressure. Any hydrazide of hyaluronic acid that will probably be formed during the reaction with hydrazinolysis is destroyed by reaction with HIO₃ (iodic acid). As the reaction may be very vigourous, it is conducted while cooling the reaction container in iced water. The product of hyrazinolysis is solubilized in 50 ml of a solution of 5% sodium acetate and reacted with 25 ml of a 0.5 M solution of iodic acid. The reaction proceeds for 30 minutes under agitation, after which 5 ml of a 57% solution of HI is added to destroy any unreacted HIO₃. The iodine that has formed is extracted from the aqueous solution with at least three 30-ml aliquots of ethyl ether (until complete decoloring of the aqueous phase). The aqueous solution is brought to neutral pH by adding a solution of NaOH 0.5 M followed by treatment with 100 ml of ethanol. The precipitate obtained is filtered with a Gooch cricible, washed with ethanol and then dried at room temperature and at reduced pressure. The product obtained is characterized analytically to determine the percentage of N-deacetylated groups and the mean molecular weight.

| | |
|---|---|
| Yield of the reaction | 90% |
| % of N-deacetylation | 26% |
| mean molecular weight | 130 Kda |

### Example 6

### Preparation of the salt of hyaluronic acid partially N-deacetylated with tetrabutylammonium (DHA/TBA)

One gram (2.5 mmol.) of hyaluronic acid sodium salt, partially N-deacetylated, is solubilized in 60 ml of water and the solution is percolated through a column filled with 25 ml of a sulfonic resin in the form of tetrabutylammonium salt (TBA). The sulphonic resin in H⁺ form is activated with a 40% solution w/v of TBAOH. The eluate, containing N-deacetylated,hyaluronic acid TBA salt is collected and freeze-dried.

### Example 7

### Preparation of p-NO₂-phenylester of benzoic acid (acylating agent)

Ten grams (0.082 mol.) of benzoic acid is solubilized in 800 ml of CH₂Cl₂, after which 11.4 g (0.082 mol.) of p-NO₂-phenol and 16.9 g (0.082 mol.) of DCC (Dicyclohexylcarbodiimide) are added. The reaction proceeds for 2 hours, while the solution is boiled and refluxed. Subsequently, the dicyclohexylurea that forms is filtered and the filtered product is dried with a rotavapor under reduced pressure. The product thus obtained is purified by repeated crystallization in ethyl acetate. The crystals are filtered and placed to dry at room temperature at reduced pressure. The derivative is characterized by TLC analysis (eluent: CH₂Cl₂/ethyl acetate 90/10 and Rf=0.77) and by IR and UV spectroscopy.

| | |
|---|---|
| Yield of the reaction | 92% |

### Example 8

### Preparation of p-NO₂-phenylester of cinnamic acid (acylating agent)

Twelve grams (0.082 mol.) of cinnamic acid is solubilized in 800 ml of CH₂Cl₂, after which 11.4 g (0.082 mol.) of p-NO₂-phenol and 16.9 g (0.082 mol.) of DCC (Dicyclohexylcarbodiimide) are added. The reaction proceeds for 2 hours, while the solution is boiled and refluxed. Subsequently, the dicyclohexylurea that forms is filtered and the filtered product is dried with a rotavapor under reduced pressure. The product thus obtained is purified by repeated crystallization in ethyl acetate. The crystals are filtered and placed to dry at room temperature at reduced pressure. The derivative is characterized by TLC analysis (eluent: CH₂Cl₂/ethyl acetate 90/10 and Rf=0.77) and by IR and UV spectroscopy.

| | |
|---|---|
| Yield of the reaction | 89% |

### Example 9

### Preparation of p-NO₂-phenylester of dodecanoic acid (acylating agent)

Sixteen grams (0.082 mol.) of dodecanoic acid is solubilized in 1 liter of CH₂Cl₂, after which 11.4 g (0.082 mol.) of p-NO₂-phenol and 16.9 g (0.082 mol.) of DCC (Dicyclohexylcarbodiimide) are added. The reaction proceeds for 2 hours, while the solution is boiled and refluxed. Subsequently, the dicyclohexylurea that forms is filtered and the filtered product is dried with a rotavapor under reduced pressure. The product thus obtained is purified by repeated crystallization in ethyl acetate. The crystals are filtered and placed to dry at room temperature at reduced pressure. The derivative is characterized by TLC analysis (eluent: CH₂Cl₂/ethyl acetate 90/10 and Rf=0.77) and by IR and UV spectroscopy.

| | |
|---|---|
| Yield of the reaction | 93% |

### Example 10

### Preparation of p-NO₂-phenylester of stearic acid (acylating agent)

23.3 grams of stearic acid is solubilized in 1 liter of CH₂Cl₂, after which 11.4 g (0.082 mol.) of p-NO₂-phenol and 16.9 g (0.082 mol.) of DCC (Dicyclohexylcarbodiimide) are added. The reaction proceeds for 2 hours, while the solution is boiled and refluxed. Subsequently, the dicyclohexylurea that forms is filtered and the filtered product is dried with a rotavapor under reduced pressure. The product thus obtained is purified by repeated crystallization in ethyl acetate. The crystals are filtered and placed to dry at room temperature at reduced pressure. The derivative is characterized by TLC analysis (eluent: CH₂Cl₂/ethyl acetate 90/10 and Rf=0.77) and by IR and UV spectroscopy.

| | |
|---|---|
| Yield of the reaction | 87% |

### Example 11

### Preparation of p-NO₂-phenylester of o-acetyl salicyclic acid (acylating agent)

14.7 grams of acetylsalicyclic acid is solubilized in 1 liter of CH₂Cl₂, after which 11.4 g (0.082 mol.) of p-NO₂-phenol and 16.9 g (0.082 mol.) of DCC (Dicyclohexylcarbodiimide) are added. The reaction proceeds for 2 hours, while the solution is boiled and refluxed. Subsequently, the dicyclohexylurea that forms is filtered and the filtered product is dried with a rotavapor under reduced pressure. The product thus obtained is purified by repeated crystallization in ethyl acetate. The crystals are filtered and placed to dry at room temperature at reduced pressure. The derivative is characterized by TLC analysis (eluent: CH₂Cl₂/ethyl acetate 90/10 and Rf=0.77) and by IR and UV spectroscopy.

| | |
|---|---|
| Yield of the reaction | 80% |

### Example 12

### Preparation of partially N-acetylated hyaluronic acid (with the benzoic acid derivative)

One gram (1.6 mmol.) of DHA/TBA (26% deacetylation) is solubilized in 50 ml of DMSO, after which 5 ml of a 10% solution of p-NO₂-phenylester of benzoic acid (prepared according to Example 7) in DMSO is added. The reaction proceeds for 24 hours, under agitation at room temperature, after which it is blocked by adding 2.5 ml of a saturated solution of NaCl. This is left to react for 30 minutes and then 100 ml of ethanol is slowly added. The precipitate thus obtained is filtered through a Gooch, washed with ethanol and ethyl ether and lastly dried at room temperature and at reduced pressure. The derivative is analyzed by TLC (after hydrolysis of the amide), colorimetric analysis of the percentage of free NH₂ groups and IR and UV spectroscopy.

| | |
|---|---|
| Yield of the reaction | 85% |
| % free NH₂ | 11% |
| % N-acylation | 15% |

### Example 13

### Preparation of partially N-acetylated hyaluronic acid (with the cinnamic acid derivative)

One gram (1.6 mmol.) of DHA/TBA (26% deacetylation) is solubilized in 50 ml of DMSO, after which 5 ml of a 10% solution of p-NO₂-phenylester of cinnamic acid (prepared according to Example 8) in DMSO is added. The reaction proceeds for 24 hours, under agitation at room temperature, after which it is blocked by adding 2.5 ml of a saturated solution of NaCl. This is left to react for 30 minutes and then 100 ml of ethanol is slowly added. The precipitate thus obtained is filtered through a Gooch, washed with ethanol/water 9:1, ethyl ether and lastly dried at room temperature and at reduced pressure. The derivative is analyzed by TLC (after hydrolysis of the amide), colorimetric analysis of the percentage of free NH₂ groups and IR and UV spectroscopy.

| | |
|---|---|
| Yield of the reaction | 85% |
| % free NH₂ | 11% |
| % N-acylation | 15% |

### Example 14

### Preparation of partially N-acetylated hyaluronic acid (with the dodecanoic acid derivative)

One gram (1.6 mmol.) of DHA/TBA (26% deacetylation) is solubilized in 50 ml of NMP, after which 5 ml of a 10% solution of p-NO₂-phenylester of dodecanoic acid (prepared according to Example 9) in NMP is added. The reaction proceeds for 24 hours, under agitation at room temperature, after which it is blocked by adding 2.5 ml of a saturated solution of NaCl. This is left to react for 30 minutes and then 100 ml of ethanol is slowly added. The precipitate thus obtained is filtered through a Gooch, washed with ethanol and ethyl ether and lastly dried at room temperature and at reduced pressure. The derivative is analyzed by TLC (after hydrolysis of the amide), colorimetric analysis of the percentage of free NH₂ groups and IR and UV spectroscopy.

| | |
|---|---|
| Yield of the reaction | 88% |
| % free NH₂ | 10% |
| % N-acylation | 16% |

### Example 15

### Preparation of partially N-acetylated hyaluronic acid (with the stearic acid derivative)

One gram (1.6 mmol.) of DHA/TBA (26% deacetylation) is solubilized in 50 ml of NMP, after which 5 ml of a 10% solution of p-NO₂-phenylester of stearic acid (prepared according to Example 10) in NMP is added. The reaction proceeds for 24 hours, under agitation at room temperature, after which it is blocked by adding 2.5 ml of a saturated solution of NaCl. This is left to react for 30 minutes and then 100 ml of ethanol is slowly added. The precipitate thus obtained is filtered through a Gooch, washed with ethanol and ethyl ether and lastly dried at room temperature and at reduced pressure. The derivative is analyzed by TLC (after hydrolysis of the amide), colorimetric analysis of the percentage of free NH₂ groups and IR and UV spectroscopy.

| | |
|---|---|
| Yield of the reaction | 85% |
| % free NH₂ | 12% |
| % N-acylation | 14% |

### Example 16

### Preparation of partially N-acetylated hyaluronic acid (with the acetyl salicylic acid derivative)

One gram (1.6 mmol.) of DHA/TBA (26% deacetylation) is solubilized in 50 ml of NMP, after which 5 ml of a 10% solution of p-NO₂-phenylester of acetyl salicylic acid (prepared according to Example 11) in NMP is added. The reaction proceeds for 24 hours, under agitation at room temperature, after which it is blocked by adding 2.5 ml of a saturated solution of NaCl. This is left to react for 30 minutes and then 100 ml of ethanol is slowly added. The precipitate thus obtained is filtered through a Gooch, washed with ethanol and ethyl ether and lastly dried at room temperature and at reduced pressure. The derivative is analyzed by TLC (after hydrolysis of the amide), colorimetric analysis of the percentage of free NH₂ groups and IR and UV spectroscopy.

| | |
|---|---|
| Yield of the reaction | 90% |
| % free NH₂ | 10% |
| % N-acylation | 16% |

### Example 17

### Preparation of benzylamide of hyaluronic acid

Two gram (3.2 mmol.) of tetrabutylammonium salt of hyaluronic acid (HA/TBA) is solubilized in 100 ml of DMSO. This solution is supplemented with 3 ml of humid acid resin in DMSO and 784 mg (4.8 mmol.) of 1,1-carbonyldiimidazole.. This is left to react under agitation for 12 hours, after which it is filtered through a Gooch crucible to eliminate the resin and the filtered product is supplemented with 1 ml (9.6 mmol) of benzylamine. This is left to react for 48 hours and then 5 ml of a saturated solution of NaCl is added and it is left under agitation for 30 minutes. It is supplemented with 200 ml of acetone and the precipitate thus obtained is filtered and dried at reduced pressure. The dry derivative is analyzed by TLC, IR and HPLC.

| | |
|---|---|
| % of amidation | 25% |

### Example 18

### Preparation of benzylamide of hyaluronic acid

Two gram (3.2 mmol.) of tetrabutylammonium salt of hyaluronic acid (HA/TBA) is solubilized in 100 ml of DMSO. The solution is adjusted to pH 3 with HCl 1 M and then 784 mg (4.8 mmol) of 1,1-carbonyldiimidazole.. This is left to react under agitation for 12 hours, after which it is filtered through a Gooch crucible to eliminate the resin and the filtered product is supplemented with 1 ml (9.6 mmol) of benzylamine. This is left to react for 48 hours and then 5 ml of a saturated solution of NaCl is added and it is left under agitation for 30 minutes. It is supplemented with 200 ml of acetone and the precipitate thus obtained is filtered and dried at reduced pressure. The dry derivative is analyzed by TLC, IR and HPLC.

| | |
|---|---|
| % of amidation | 15% |

### Example 19

### Preparation of benzylamide of hyaluronic acid

Two gram (3.2 mmol.) of hyaluronic acid in acid form is solubilized in 100 ml of DMF. To this solution is is added 854 mg (5.2 mmol.) of 1,1-carbonyldiimidazole. This is left to react under agitation for 6 hours, after which 1.13 ml (10.4 mmol) of benzlyamine is added. The reaction proceeds for 48 hours and then is blocked by adding 200 ml of acetone. The precipitate obtained is filtered and dried under reduced pressure. The dry derivative is characterized by TLC, IR and HPLC.

| | |
|---|---|
| % of amidation | 60% |

### Example 20

### Preparation of benzylamide of hyaluronic acid

Two gram (3.2 mmol.) of hyaluronic acid in acid form is solubilized in 100 ml of DMF. To this solution is is added 2 ml of pyridine, 3.68 g (0.025 mmol.) of p-NO₂-phenol and pyridine chloride until a pH of 7/8 is reached. Lastly, 5.3 (0.026 mol.) of DCC and 2.8 (0.026 mol.) of benzylamine are added. This is left to react under agitation for 16 hours, after which the is blocked by adding 200 ml of acetone. The precipitate obtained is filtered and dried under reduced pressure. The dry derivative is characterized by TLC, IR and HPLC.

| | |
|---|---|
| % of amidation | 5% |

### Example 21

### Preparation of benzylamide of hyaluronic acid

Two gram (3.2 mmol.) of HA/TBA is solubilized in 100 ml of DMSO. The solution is insufflated with gaseous HCl until the reaction mixture reaches a pH of between 4.5 and 5. Subsequently, 518 mg (3.2 mmol.) of carbonyldiimidazole is added. This is left to react under agitation for one hour, after which 0.700 ml (6.4 mmol.) of benzylamine is added. The reaction proceeds for 16-18 hours. After this time, 5 ml of a saturated solution of NaCl is added. It is precipitated by adding 200 ml of acetone and the precipitate thus obtained is filtered and dried at reduced pressure. The dry derivative is analyzed by TLC, IR and HPLC.

| | |
|---|---|
| % of amidation | 50% |

### Example 22

### Preparation of octylamide of hyaluronic acid

Two gram (3.2 mmol.) of HA/TBA is solubilized in 100 ml of DMSO. The solution is insufflated with gaseous HCl until the reaction mixture reaches a pH of between 4.5 and 5. Subsequently, 207 mg (1.28 mmol.) of carbonyldiimidazole is added. This is left to react under agitation for one hour, after which 0.417 ml (3.2 mmol.) of octylamine is added. The reaction proceeds for 16-18 hours. After this time, 5 ml of a saturated solution of NaCl is added. It is precipitated by adding 200 ml of acetone and the precipitate thus obtained is filtered and dried at reduced pressure. The dry derivative is analyzed by TLC, IR and HPLC.

| | |
|---|---|
| % of amidation | 25% |

### Example 23

### Preparation of dodecyl amide of hyaluronic acid

Two gram (3.2 mmol.) of HA/TBA is solubilized in 100 ml of DMSO. The solution is insufflated with gaseous HCl until the reaction mixture reaches a pH of between 4.5 and 5. Subsequently, 104 mg (0.64 mmol.) of carbonyldiimidazole is added. This is left to react under agitation for one hour, after which 600 mg (3.2 mmol.) of dodecylamine is added. The reaction proceeds for 16-18 hours. After this time, 5 ml of a saturated solution of NaCl is added. It is precipitated by adding 200 ml of acetone and the precipitate thus obtained is filtered and dried at reduced pressure. The dry derivative is analyzed by TLC, IR and HPLC.

| | |
|---|---|
| % of amidation | 15% |

### Example 24

### Preparation of hexadecylamide of hyaluronic acid

Two gram (3.2 mmol.) of HA/TBA is solubilized in 100 ml of DMSO. The solution is insufflated with gaseous HCl until the reaction mixture reaches a pH of between 4.5 and 5. Subsequently, 52 mg (0.32 mmol.) of carbonyldiimidazole is added. This is left to react at room temperature under agitation for one hour, after which 780 mg (3.2 mmol.) of hexadecylamine is added. The reaction proceeds for 16-18 hours. After this time, 5 ml of a saturated solution of NaCl is added. It is precipitated by adding 200 ml of acetone and the precipitate thus obtained is filtered and dried at reduced pressure. The dry derivative is analyzed by TLC, IR and HPLC.

| | |
|---|---|
| % of amidation | 5% |

### Evaluation of Biological Properties

In this study human preadipocytes were isolated and cultured. 10⁶ preadipocytes were seeded onto different scaffolds and implanted in 42 nude mice to test new materials. Sponges and nonwoven materials based on hyaluronic acid modified by esterification (HYAFF®11) and collagen sponges were used. Scaffolds without cells served as negative controls in the same animal. After 3 and 8 weeks the grafts were explanted. Macroscopical appearance, weight, thickness, microscopy, immunohistochemistry and TEM (scaffold structure, cellularity, penetration depth of the seeded cells, vascularization) were assessed and evaluated for differences in scaffold-cell interactions.

**Results:** In vitro preadipocytes differentiated earlier when attached to HYAFF® 11 scaffolds. Macroscopically all preadipocyte-constructs appeared yellowish and presented numerous vessels, the controls appeared white and avascular. Microscopically HYAFF®11 constructs showed higher cell densities than collagen constructs. The pores of the sponges contained more differentiated adipocytes than the nonwoven, while the undifferentiated preadipocytes were more numerous in the nonwoven. Penetration of adipose precursor cells was deeper and more homogenous in HYAFF®11 scaffolds. All preadipocyte grafts had better vascularization than the controls; vessel formation was more pronounced around mature adipocytes. Electron microscopy demonstrated well differentiated adipocytes and large amounts of ECM around preadipocytes in HYAFF®11 sponges.

**Conclusion:** In vitro cultured human preadipocytes differentiate into adipose-like tissue. This promising method will be used for future reconstruction of soft tissue defects. HYAFF®11 sponges supported the expansion and differentiation of the adipose precursor cells. This carrier is superior to the nonwoven with regard to adipocyte differentiation and superior to the collagen sponge with regard to cellularity.

### Materials and Methods

### Scaffolds

### Collagen sponges (CS)

Collagen sponge scaffolds were produced by a directional solidification method described by Heschal et al., Possible applications of directional solidification techniques in cryobiology. In P. Kittel (Ed.) *Advances of Cryogenic Engineering.* Vol. 41, New York: Plenum Press, 1996. Bovine collagen type I (1.8 wt.%) suspension (Dr. Otto Suwelack GmbH, Germany) was frozen and subsequent freeze-dryed, see WO 99/27315. The remaining pores correspond to the previous ice crystal structure with an average pore size of 50 µm as described by Schoof et al., Einfluß des Einfriervorganges auf die Porenstruktur gefriergetrockneter Kollagenschwämme. *Ki-Luft und Kältetechnik* 34, 1998.

### HYAFF®11 sponges (HS)

HYAFF®11 (a linear derivative of hyaluronic acid is modified by complete esterification of the carboxylic function of the glucuronic acid with benzyl groups) sponges were prepared as described above and by Rastrelli et al., Hyaluronic acid esters, a new class of semisynthetic biopolymers: chemical and physico-chemical properties. *Clin. Implant. Mater*. 9: 199, 1990. HS structure has open interconnecting pores obtained by an technology which coupled a phase inversion process with a low-pressure gas process. The pore size varies between 50 to 340 µm.

### HYAFF®11 nonwoven (HV)

HV is composed of nonwoven fibers (20 µm thick) of hyaluronan benzyl ester with a specific weight of 100 g/m² which was also prepared as described above.

For the experiments all sterilized materials were cut into samples (0.75cm x 0.75cm x 0.5cm).

### Cell cultures and biohybrids in vitro

Preadipocytes were isolated out of freshly excised human subcutaneous adipose tissue (0.4-0.7 grams) of young adults (age: 18-29 years) at the Department of Plastic Surgery and Hand Surgery - Burn Center who underwent elective operations (e.g. reduction mammaplasty). The fibrous tissue was removed, the adipose tissue was minced into pieces and digested by collagenase 0.1 U ml⁻¹/dispase 0.8 U ml⁻¹ (Boehringer Mannheim, Germany) in a water bath at 37°C for 60 min. under permanent shaking. The digestion was stopped by adding Dulbecco's modified Eagle medium (DMEM) containing 15% FCS (Biochrom, Berlin, Germany) and incubated in erythrocyte lysis buffer (154mmol l⁻¹ NH4Cl, 10mmol l⁻¹ KHCO₃, 1mmol l⁻¹ EDTA, 10 minutes). The cell suspension was centrifuged (200xg at 17°C for 10 min.) and the cells were seeded on tissue culture dishes (63.6 cm², Greiner, Solingen, Germany) with DMEM 15% FCS (added 100 U ml⁻¹ Penicillin, 100µg ml⁻¹ Streptomycin) with a seeding density of 3x10⁴ cells/cm². The cells were cultured at 37°C at 10% CO₂, medium was changed on day 2 and supplemented with EGF (epidermal growth factor, 10ng ml⁻¹, Sigma). Preadipocytes of the second passage at confluence were trypsinized, resuspended and counted in a hemocytometer. A suspension of 100µl containing 1 x 10⁶ ± 5 x 10⁴ cells (preadipocyte pools) was seeded on the upper surface by gentle dropping on the FCS-wetted (FCS soaking of the scaffolds lastet for 24 hours at 37°C prior the seeding of the cells) and left in the incubator for 24 hours to allow cell attachment.

### In vivo experimental model

Nude athymic mice (8-week-old, NMRI nu/nu) were operated under aseptic conditions and inhalational anaesthesia (Enflurane®). 42 fabricated preadipocyte/scaffold (+) constructs and 42 negative (-) controls (scaffold without cells, 24 hrs. soaked with DMEM) were transplanted. Every animal received one preadipocyte/scaffold construct subcutaneously to the left scapular area and its matching control to the contralateral side through separate incisions. The bottom of the constructs was placed on the muscle fascia. All animal experiments had been performed according to the German law on the Protection of Animals. After 3 weeks (group A, 21 animals carrying 7 CS+ and 7 CS-, 7 HS+ and 7 HS-, 7 HV+ and 7 HV-) and 8 weeks (group B, 21 animals 7 CS+ and 7 CS-, 7 HS+ and 7 HS-, 7 HV+ and 7 HV-) the mice were killed by overdose of gaseous anesthetic. The specimens were removed for macroscopic (colour, vessels, ingrowth) and microscopic analysis. The weight of each sponge was assessed prior to transplantation and after explantation.

### Histology and Immunohistochemistry

One part of the vertically bisected specimens was fixed in 4% buffered formaldehyde solution and later embedded in paraffin, the other half was cryofixed. Both were vertically sectioned. Ultrastructural images were obtained from in vitro (3 specimens) and in vivo specimens (6 specimens). Tissue slices of 6 µm (paraffin specimens) were prepared and dyed with hematoxylin-eosin and Giemsa. The cryofixed fragments were stained with oil-red for identification of lipid vacuoles. Paraffin sections of the seeded matrices and unseeded controls were stained by monoclonal antibodies specific for human vimentin (mahv, clone V9, Code Nr. M 0725 Lot 057, DAKO, Denmark) at a dilution of 1:10. Three examiners, who did not know to which group the sections belonged, independently assessed the histological sections. When there were differences between both assessments, the mean value was calculated.

*Non-specific cellularity* (donor and host) was assessed by counting all Giemsa stained cell nucleus in 5 defined microscopic fields of the cross-sections at 200x magnification.

*Specific cellularity* (donor = human) was evaluated by counting all human-vimentin positive cells in 5 defined microscopic fields at 200x magnification. *Penetration depth of donor cells* was measured in 3 defined microscopic fields at 200x magnification by using intraocular micrometer (Zeiss).

*Vascularization of the grafts* was estimated in the cross-sections. In case of no vessels "-" was given, vessels in one ore more surface regions were rated "+", vessels in the central region "++" and a homogenous distribution of vessels within the graft "+++" was given.

*Ultrastructure* of the grafts was assessed after postfixation processing and viewed by a Philips EM 400 electron microscope.

### Statistical evaluation

Data of the weight and thickness of the grafts, the overall cellularity in the grafts and the penetration depth of the seeded human preadipocytes were expressed as mean value and ± standard deviation. The significance of differences between different implantation periods and between the preadipocyte/scaffold constructs and the negative controls was evaluated by Wilcoxon signed rank test. Differences at p < 0.05 were regarded to be significant.

### Results:

### Culture

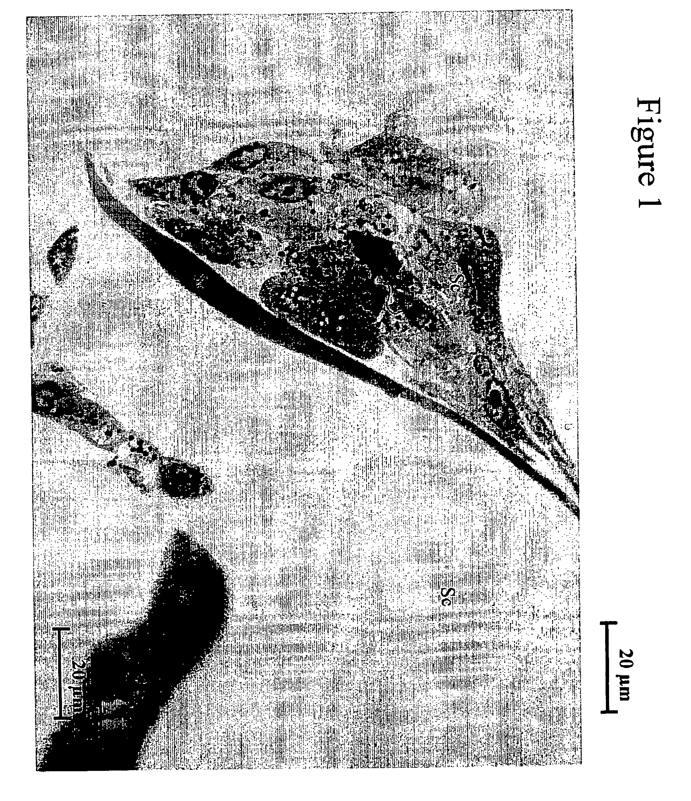

The cells well adhered to the scaffolds 24 hours after the seeding (Fig. 1). Penetration of the cells in vitro was only observed in the scaffold surface areas. Immediately before transplantation, the adipose precursors in HS+ and HV+ showed some cytoplasmic vacuoles and round shape as signs of differentiation (Fig. 1) in histology and in ultrastructure. This was not observed in CS+ specimens.

### Gross morphology

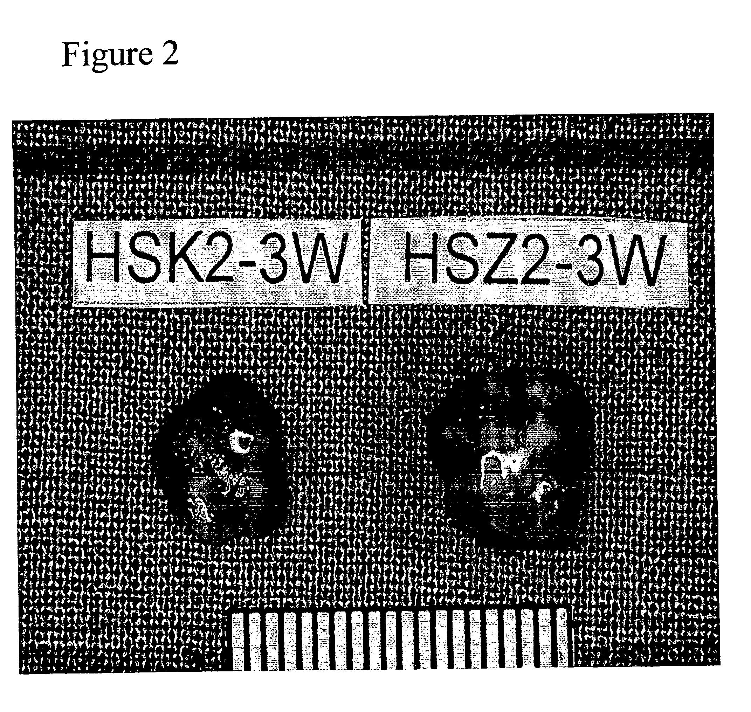

All specimens were easily identified. The gross shape of the CS+ and HS+ specimens was almost unchanged after 3 and 8 weeks. CS- and HS- specimens showed round edges and looked smaller than CS+ and HS+. HV+ showed great variation between the specimens, HV- scaffolds showed the highest deformation and shrinkage. All preadipocyte/scaffold contructs were covered by tightly adherent layers of macroscopically yellow tissue and new vessels on the top (Fig. 2). The control grafts appeared white and almost avascular (Fig. 2).

### Weight changes as a function of time (Table 1)

*CS+ and CS- weights:* After 24 hours in vitro there was no difference in weight between the collagen scaffolds carrying preadipocytes CS+ and those without cells CS-. After 3 weeks in vivo there was significant weight loss for CS+ and CS-. CS+ presented a significant higher weight than CS-. The weight reduction between week 3 and 8 was lower than the weight reduction between implantation and week 3.
*HS*+ *and HS*- *weights:* After 24 hours in vitro the weight of preadipocyte/scaffold constructs HS+ was higher than the controls HS-. After 3 weeks in the nude mouse there was an increase in weight in HS+ while there was a weight reduction in the controls HS-. The difference was significant. After 8 weeks HS+ still had a significant higher weight than the controls.

*HV+ and HV- weights:* The nonwoven matrix with preadipocytes showed higher weights than the controls at all times. A slight weight gain with a remarkable variation in HV+ was seen after 8 weeks in vivo.

Comparing the three scaffolds (CS, HS and HV) with each other, HV showed the lowest weight of the scaffolds examined and the greatest variation between the specimens. The grafts with preadipocytes had higher weight in all grafts at all times, this was significant for HS+ and HV+. A significant weight gain was only observed in HS+ carrying preadipocytes which corresponded to the adipose-like tissue which developed in this scaffold.

### Histomorphologic chronology of the grafts:

### Overview

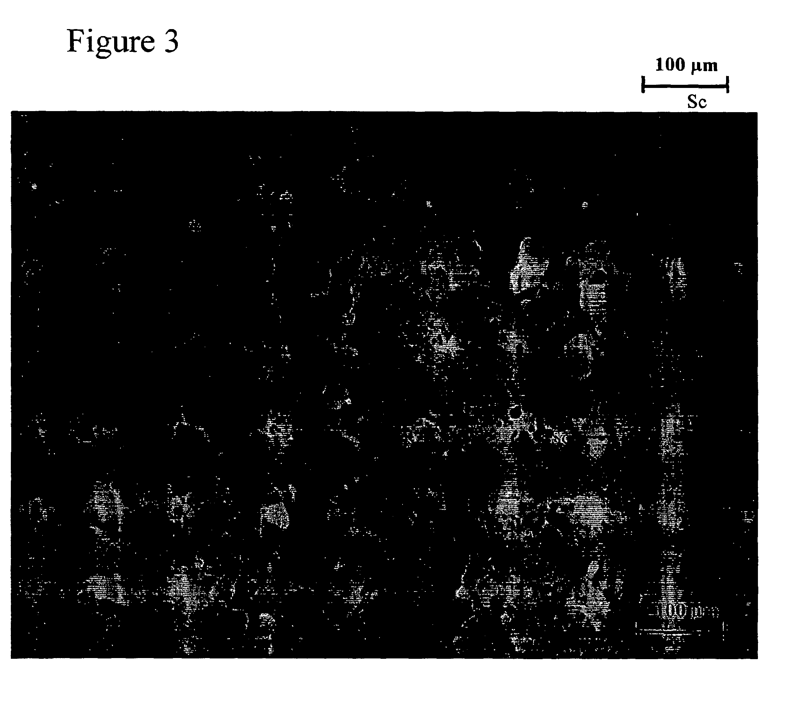

Every explanted specimen were surrounded by thin fibrous capsule, which separated the scaffold with the newly formed adipose-like tissue from surrounding host tissue. Microscopical examination of the preadipocyte/scaffold constructs demonstrated viable adipose tissue located on the surfaces of the scaffolds under the capsule. Mature adipocytes were found in the areas beneath the surface. No differentiated adipocytes were observed in the central region of the sponges. Many vessels presented in this new formed adipose tissue. In the control grafts no adipocytes were found at all. The pores appeared to have collapsed in HV (swollen fibers and narrowed pores) and in CS (infractured areas and obstructed pores). Only in HS the porous structure well sustained. In these HS+ areas adipose tissue was composed of clusters of adipocytes (Fig. 3).

### Cellularity

The non-specific (donor and host) cellularity in collagen specimens was greater in the CS+ grafts than in the CS- after 3 and after 8 weeks while there was a reduction of the non-specific cellularity in CS+ and CS- (Table 2).
In the HYAFF 11 specimens there was a higher non-specific cellularity in the control grafts (HS- and HV-) than in the preadipocyte carrying specimens (HS+ and HV+). Between week 3 and week 8 there was a slight reduction of the non-specific cellularity in HS+, HV+ and HV- (Table 2).

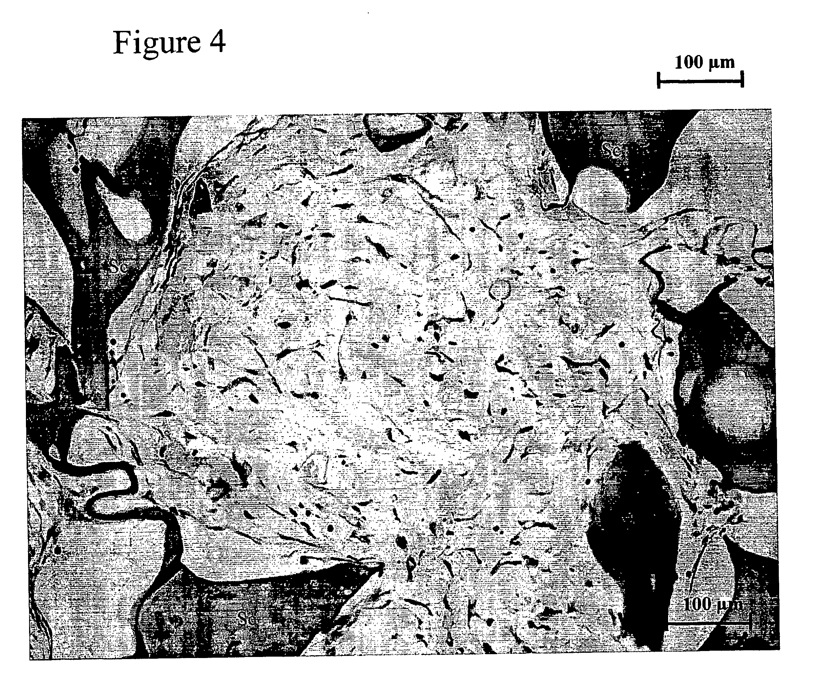

Staining for human-vimentin indicated that cells in CS+, HS+ and HV+ specimens were strongly positive demonstrating human origin (Fig. 4). There was no positive staining in the CS-, HS- and HV- specimens. This specific (donor) cellularity showed interesting results in the three different scaffolds (Table 2). In HS+ there was no difference between week 3 and week 8; the non-specific cellularity was higher than the specific cellularity. In CS+ there was a slight reduction between week 3 and week 8. In HV+ there were significant differences between week 3 and week 8. The highest specific cellularity of all scaffolds was observed in HV+ at week 3 (115 cells/field) but 8 weeks after implantation the number of human cells was reduced in HV+ to the lowest number of all scaffolds (26 cells/field).

### Penetration depth

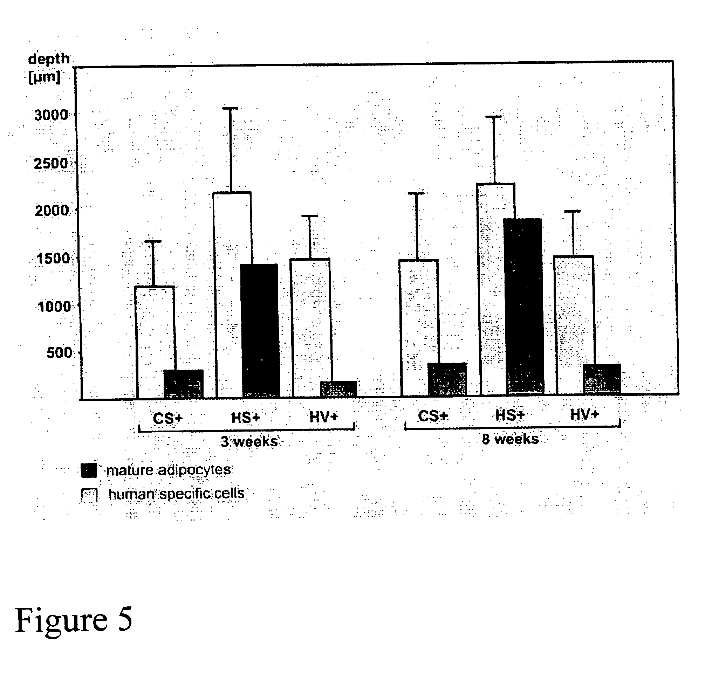

Penetration depth of the human adipose precursor cells after 3 weeks in vivo was different for each of the materials (Table 3, Fig. 5). Best penetration was found in HS+. After 3 weeks most HS+ specimens presented complete penetration of the cells, after 8 weeks in all HS+ the penetration was complete. The penetration depth was ¾ of the scaffold thickness in HV+ after 3 weeks and complete in HV+ after 8 weeks. In CS+ there was partial penetration after 3 weeks with increasing depth, but still partial, after 8 weeks.

Mature adipocytes were only found in some areas. The highest number of mature adipocytes penetrated 1800 µm in HS+ (Fig. 3), the least number of mature adipocytes penetrated only 280 µm in HV+ (Table 3, Fig. 5). No adipocytes were found in any of the control grafts.

### Neovascularization

There was remarkably better new vessel formation in the preadipocyte/scaffold (+ Median) constructs compared to the negative controls (-Median) for all materials (Table 4). In the open interconnecting pores of HS the neovascularization was best (++). In none of the explanted grafts was a homogenous and rich vessel distribution (+++).

### Ultrastructure of the grafts:

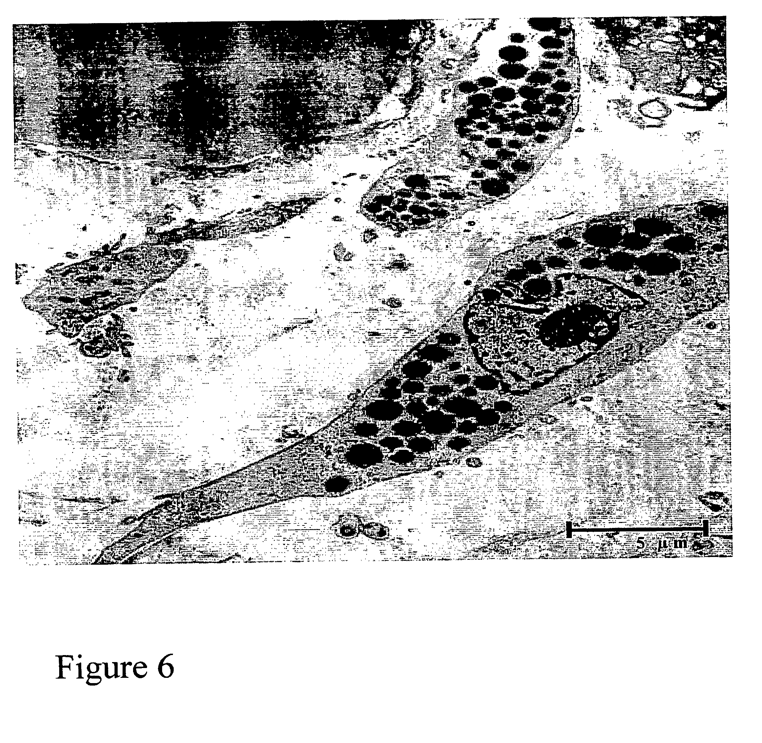

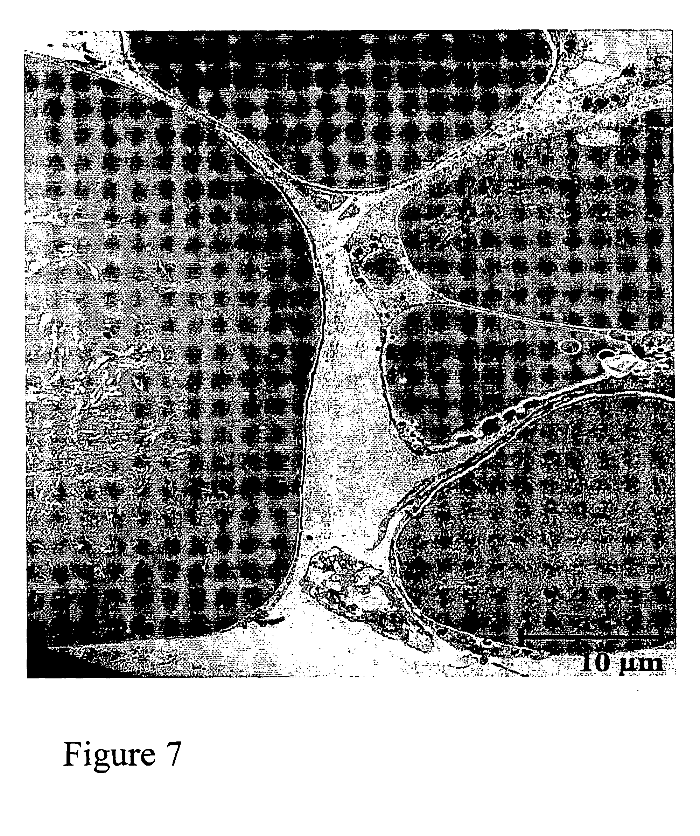

Electron microscopy detailed human adipose precursor cells and their interaction with the scaffolds. After 3 weeks in vivo numerous cytoplasmic lipid droplets were found in all preadipocytes. In HV+ mainly spindle-shape preadipocytes with multiple small lipid droplets were found (Fig. 6) indicating no complete differentiation. Undifferentiated fibroblast-like precursors presented deep inside the nonwoven (HV+) in narrow pores closely attached to the carrier material. In HV-much more cells were found after 3 weeks than in the preadipocyte/scaffold constructs. Many histiocytes and giant cells occured in the negative control HV-grafts. In HS+ mainly round-shape cells with single large lipid droplets with adipocyte-typical signet ring appearance were found (Fig. 7). There were multiple capillaries located near the preadipocytes and adipocytes. New collagen fibrils were found in close vicinity to the scaffold. Adipocytes were closely attached to the scaffold and bundles of new collagen fibrils were found inbetween (Fig. 7). After 8 weeks in vivo morphology of the adipocytes was similar to the 3 weeks specimens. The scaffolds showed a more irregular structure and obstructed pores by collapsed scaffold remnants. There were some giant cells and newly formed ECM mainly composed of collagen fibers.

**Table 1:**

| Mean weights of the different scaffolds in vitro (24 hours) and after being implanted in nude mice with (+) and without (-) attached human preadipocytes. CS (collagen sponges), HS (HYAFF®11 sponges) and HV (HYAFF®11 nonwoven). | | | |
|---|---|---|---|
| Weights (g) | 24 hrs in vitro | 3 weeks in vivo | 8 weeks in vivo |
| CS+ | 0.395 ±0.046 | 0.195 ±0.013*,** | 0.168±0.025 |
| CS- | 0.396 ±0.067 | 0.166 ±0.016* | 0.159 ±0.014 |
| HS+ | 0.209 ±0.025 | 0.24 ± 0.041**** | 0.232±0.047**** |
| HS- | 0.166 ±0.012 | 0.133 ±0.017*** | 0.171 ±0.07 |
| HV+ | 0.127 ±0.018 | 0.103 ±0.011***** | 0.167 ±0.117 |
| HV- | 0.101 ±0.035 | 0.072 ±0.007***** | 0.121±0.054 |

| | | | |
|---|---|---|---|
| * After 3 weeks in vivo there was significant (p<0.05) weight loss for both, CS+ and CS-. CS+ had a significant higher weight **(p<0.05) compared to CS-. | | | |
| *** After 3 weeks in the nude mouse there was an increase in weight in HS+ while there was a significant weight reduction in the controls HS- (p<0.05) compared to in vitro weight. | | | |
| **** At both timepoints (after 3 and 8 weeks) HS+ has a significant higher weight than the controls HS- (p<0.05). | | | |
| ***** After 3 weeks both HV+ and HV- had a significant lower weight than at the time of implantation. | | | |

**Table 2:**

| Cellularity (mean) in the different scaffolds implanted in nude mice with (+) and without (-) attached human preadipocytes. Non-specific includes all cells stained by Giemsa (donor and host), specific includes all cells of human origin stained by MAH-Vim (donor). | | | | |
|---|---|---|---|---|
| Cellularity (cells/field) | 3 weeks in vivo | | 8 weeks in vivo | |
| | non-specific | specific | non-specific | specific |
| CS+ | 97±49 | 60±14 | 77±56 | 40±6 |
| CS- | 80±45 | | 66±44 | |
| HS+ | 95±45 | 52±27 | 87±45 | 53±26 |
| HS- | 120±69 | | 132±43 | |
| HV+ | 195±68 | 115±56 | 134±59 | 27±22 |
| HV- | 210±129 | | 145±66 | |

**Table 3:**

| Penetration depth (mean) of undifferentiated human preadipocytes stained by MAH-Vim in the different preadipocyte/scaffolds constructs implanted in nude mice. Differentiated adipocytes stained by Oil-red were only found in some areas, the maximum depth is given. | | | | |
|---|---|---|---|---|
| Penetration depth (µm) | 3 weeks in vivo | | 8 weeks in vivo | |
| | preadipocytes | adipocytes | preadipocytes | adipocytes |
| CS+ | 1188±489 | 290 | 1433±686 | 350 |
| HS+ | 2158±897 | 1400 | 2227±706 | 1800 |
| HV+ | 1460±447 | 170 | 1446±482 | 280 |

**Table 4:**

| Neovascularization in different scaffolds implanted in nude mice with (+) and without (-) attached human preadipocytes. "-" no vessels, "+" vessels in one or more surface regions, "++" vessels in central regions, "+++" homogenous vessel distribution (median). | | | | | | |
|---|---|---|---|---|---|---|
| Neovascularization | CS+ | CS- | HS+ | HS- | HV+ | HV- |
| 3 weeks in vivo | + | - | ++ | + | + | - |
| 8 weeks in vivo | + | - | + | + | + | - |

Tissue-engineered adipose tissue has the potential to correct congenital, idiopathic or traumatic soft tissue defects in all areas of the body without creating a major donor defect. The present study demonstrates the feasibility of engineering an adipose-like tissue by transplanting cultured preadipocytes followed by a differentiation after implantation.

In 3 week and 8 week in vivo studies, hyaluronan benzyl ester (HYAFF®11) sponge proved a better scaffold than collagen sponge and hyaluronan benzyl ester (HYAFF®11) nonwoven with respect to constant weight, homogenous distribution of survived donor precursor cells and finally the highest amount of differentiated adipose tissue. The hyaluronan benzyl ester sponge not only showed a constant weight but also the highest weight of all scaffolds investigated. Constant weight and size of a soft tissue filler material are the most important criterias for clinical use.

The initial specific cellularity (after 3 weeks in vivo) on hyaluronan benzyl ester nonwoven (HV+) was twice as high as that on collagen (CS+) and hyaluronan sponges (HS+). The final specific cellularity per field (after 8 weeks in vivo) on sponges (CS+, HS+) was twice as high as that on nonwoven (HV+). The specific cellularity indicates the seeded human mesenchymal precursor cells. It is likely that collapse of the nonwoven (swollen fibers and narrowed pores) decreased the space available for the preadipocytes to differentiate. Infractured areas and obstructed pores of the collagen scaffold after 8 weeks did not show any differentiated adipocytes (CS+). In hyaluronan benzyl ester sponges (HS+) the porous structure was still present after 8 weeks and in these areas adipose tissue was composed of clusters of adipocytes.

All explants with attached human preadipocytes appeared vascular, as compared to control scaffolds without cells. The controls looked white and almost avascular. This result is probably attributed to extracellular matrix produced by cultured adipose precursors which is potent inducer of neovascularization. The vessels within the specimens confirmed the gross morphology. Neovascularization was much more pronounced in preadipocyte/scaffold (+) constructs.

Non-specific cellularity in hyaluronan benzyl ester scaffolds without adipose precursor cells (HS-, HV-) was higher than that in HS+ and HV+. Histiocytes and multi-nucleated giant cells were observed in higher number in the negative controls indicating that tissue (foreign body) reaction was much more intense. These observations are contradictory to the non-specific cellularity in collagen scaffolds. These results cannot be explained by our todays knowledge. It might be attributed to preadipocyte secretion of inhibitory factor when grown and transplanted on hyaluronan benzyl ester material. This observation need to be examined in further experiments.

Penetration depth of cells into a matrix is a good parameter for scaffold/cell interaction analysis in tissue engineering. In the present study human cells were still present after 8 weeks in the nude mouse in all scaffold types. There was increasing penetration of the cells over this time period in sponges. These biodegradable hyaluronic acid spongy carriers supported the expansion and differentiation of the precursor cells. A more homogenous distribution of the seeded cells was leading to a less concentrated number in the examined fields connected with a better penetration into the graft.

Human preadipocytes penetrated the scaffold in vivo to a mean depth of 2227 ±706µm in HS+, mature adipocytes were found to a mean depth of 520µm (maximum 1800µm). We conclude that preadipocytes need enough porous space to differentiate to mature adipocytes. The optimal material and scaffold architecture used in adipose tissue engineering need to be defined. The ideal scaffold, according to the present results contains large (>120µm) interconnected pores and has reduced swelling characteristics. The HYAFF®11 sponge proved to be superior to the HYAFF®11 nonwoven and the collagen sponge in the present experiments.

A new strategy for applied clinical research should be the reconstruction in vitro of tissues and entire organs obtained by guided in vitro proliferation of autologous cells of the same patient. The three-dimensional structure of a scaffold carrying mesenchymal cells is important for the deposition of extracellular matrix. The quality of the matrix is important for the regulation of cellular activities and for the quality of the extracellular matrix to achieve similar properties of the substitute as the native tissue. Therefore it is important to investigate the influence of different matrices on the vitro and in vivo behavior of the seeded cells. Many materials have been investigated in the field of tissue engineering over the last decade. Collagen-based scaffolds as one of the first biodegradable carriers and hyaluronan-based scaffolds as a new material in tissue engineering were examined in the present study. The porous collagen matrix can support cellular ingrowth and new matrix synthesis. Collagen scaffold provides a good matrix to fibroblasts which fully differentiate and exhibit normal morphology and metabolism in vivo. Hyaluronic acid is present in the extracellular matrix of many tissues and abundant in mesenchymal tissue in the fetus and it is believed that HA based biomaterials are supportive for progenitor cell development and will facilitate tissue repair. During adipocyte differentiation precursor cells acquire characteristics of adipocytes and drastic changes occur in cell morphology. The physical connection between extracellular matrix and the nuclear matrix on one side and the quality of the extracellular matrix on the other side influence mainly adipocyte differentiation. ECM of the loose connective tissue of the native adipose tissue interconnects the adipocytes and induces fat cell clustering in vivo. It is therefore concluded that hyaluronan which is present in all the connective tissues positively influences the adipose conversion in vivo. Since clusters of mature adipocytes were only found in HYAFF® 11 sponges and not in HYAFF®11 nonwoven it is concluded that the quality of the scaffold is as important as the architecture. In the present study hyaluronan benzyl ester spongy scaffolds gave the best results of the materials investigated.

## Claims

1. A biomaterial for soft tissue reconstruction comprised of
(a) a support material comprised of a benzyl ester of hyaluronic acid; and
(b) preadipocyte cells seeded on said support material.

2. A biomaterial according to claim 1, further comprising at least one member of the group consisting of mesenchymal stem cells and endothelial cells.

3. A biomaterial according to claim 1 or 2, wherein said benzyl ester of hyaluronic acid is a 100% ester, wherein all of the carboxyl groups of said hyaluronic acid are esterified with a benzyl alcohol residue.

4. A biomaterial according to claim 1 or 2, wherein said benzyl ester of hyaluronic acid is a 5 to 99% ester, wherein 5 to 99% of the carboxyl groups of said hyaluronic acid are esterified with a benzyl alcohol residue and the remaining groups are salified.

5. A biomaterial according to any one of claims 1-4, wherein said support material is a scaffold in the form of a spongy or non-woven material.

6. A biomaterial according to any one of claims 1-5, wherein at least one member of said group of preadipocyte cells, mesenchymal stem cells and endothelial cells are human cells.

7. A biomaterial according to any one of claims 1-6 for treating soft tissue damage.

8. A biomaterial according to any one of claims 1-7 for reconstructive treatment of soft tissue damage.

9. An injectable preparation for filling soft tissue defects and depressions comprised of
(a) a totally water soluble hyaluronic acid derivative or a partially water soluble hyaluronic acid derivative and
(b) preadipocyte cells suspended in the preparation.

10. The injectable preparation according to claim 9, further comprising at least one member of the group consisting of mesenchymal stem cells and endothelial cells.

11. An injectable preparation according to claim 9 or 10, wherein the totally water-soluble hyaluronic acid derivative is an amide of hyaluronic acid.

12. An injectable preparation according to claim 9 or 10, wherein the totally water-soluble hyaluronic acid derivative is a dodecyl amide of hyaluronic acid.

13. An injectable preparation according to claim 9 or 10, wherein the partially water-soluble hyaluronic acid derivative is a 5 to 99% ester, wherein 5 to 99% of the carboxyl groups of said hyaluronic acid are esterified with a benzyl alcohol residue and the remaining groups are salified.

14. An injectable preparation according to claim 9 or 10, wherein the partially water-soluble hyaluronic acid derivative is an 85% ester, wherein 85% of the carboxyl groups of said hyaluronic acid are esterified with a benzyl alcohol residue and the remaining groups are salified.

15. An injectable preparation according to claim 9 or 10, wherein at least one member of said group of preadipocyte cells, mesenchymal stem cells and endothelial cells are human cells.

16. A preparation according to any one of claims 9-15 for filling soft tissue defects and depressions.

17. A preparation according to any one of claims 9-16 for filling soft tissue defects, depressions, wrinkles and deformities of a patient in need thereof.

18. Biomaterial according to any one of claims 1-5 or injectable preparation according to any one of claims 9-15 for use in the reconstructive treatment of soft tissue damage.

## Patentansprüche

1. Biomaterial zur Bindegewebe-Rekonstruktion, umfassend:
(a) ein Trägermaterial, das einen Benzylester von Hyaluronsäure umfasst, und
(b) Preadipozytenzellen, die auf dem Trägermaterial ausgesät sind.

2. Biomaterial nach Anspruch 1, das außerdem mindestens ein Glied der Gruppe, die aus mesenchymalen Stammzellen und Endothelzellen besteht, umfasst.

3. Biomaterial nach Anspruch 1 oder 2, wobei der Benzylester von Hyaluronsäure ein 100%-Ester ist, in dem alle Carboxylgruppen der Hyaluronsäure mit einem Benzylalkoholrest verestert sind.

4. Biomaterial nach Anspruch 1 oder 2, wobei der Benzylester von Hyaluronsäure ein 5- bis 99%-Ester ist, in dem 5 bis 99% der Carboxylgruppen der Hyaluronsäure mit einem Benzylalkoholrest verestert sind und die restlichen Gruppen ein Salz gebildet haben.

5. Biomaterial nach einem der Ansprüche 1 bis 4, wobei das Trägermaterial ein Gerüst in Form eines schwammartigen oder "non-woven"-Materials ist.

6. Biomaterial nach einem der Ansprüche 1 bis 5, wobei mindestens ein Glied der Gruppe aus Preadipozytenzellen, mesenchymalen Stammzellen und Endothelzellen humane Zellen sind.

7. Biomaterial nach einem der Ansprüche 1 bis 6 zur Behandlung einer Bindegewebsschädigung.

8. Biomaterial nach einem der Ansprüche 1 bis 7 zur rekonstruktiven Behandlung einer Bindegewebsschädigung.

9. Injizierbares Präparat zum Auffüllen von Bindegewebsdefekten und -depressionen, umfassend
(a) ein vollständig wasserlösliches Hyaluronsäurederivat oder ein partiell wasserlösliches Hyaluronsäurederivat und
(b) Preadipozytenzellen, die in dem Präparat suspendiert sind.

10. Injizierbares Präparat nach Anspruch 9, das außerdem mindestens ein Glied aus der Gruppe, bestehend aus mesenchymalen Stammzellen und Endothelzellen, umfasst.

11. Injizierbares Präparat nach Anspruch 9 oder 10, wobei das vollständig wasserlösliche Hyaluronsäurederivat ein Amid von Hyaluronsäure ist.

12. Injizierbares Präparat nach Anspruch 9 oder 10, wobei das vollständig wasserlösliche Hyaluronsäurederivat ein Dodecylamid von Hyaluronsäure ist.

13. Injizierbares Präparat nach Anspruch 9 oder 10, wobei das partiell wasserlösliche Hyaluronsäurederivat ein 5- bis 99%-Ester ist, wobei 5 bis 99% der Carboxylgruppen der Hyaluronsäure mit einem Benzylalkoholrest verestert sind und die restlichen Gruppen ein Salz gebildet haben.

14. Injizierbares Präparat nach Anspruch 9 oder 10, wobei das partiell wasserlösliche Hyaluronsäurederivat ein 85%-Ester ist, wobei 85% der Carboxylgruppen der Hyaluronsäure mit einem Benzylalkoholrest verestert sind und die restlichen Gruppen ein Salz gebildet haben.

15. Injizierbares Präparat nach Anspruch 9 oder 10, wobei mindestens ein Glied der Gruppe aus Preadipozytenzellen, mesenchymalen Stammzellen und Endothelzellen humane Zellen sind.

16. Präparat nach einem der Ansprüche 9 bis 15 zum Auffüllen von Bindegewebsdefekten und -depressionen.

17. Präparat nach einem der Ansprüche 9 bis 16 zum Auffüllen von Bindegewebedefekten, -depressionen, -falten und Missbildungen bei einem Patienten, der dessen bedarf.

18. Biomaterial nach einem der Ansprüche 1 bis 5 oder injizierbares Präparat nach einem der Ansprüche 9 bis 15 zur Verwendung bei der rekonstruktiven Behandlung von Bindegewebsschädigung.

## Revendications

1. Matériel biologique pour la reconstruction de tissus mous, constitué de
(a) un matériau support constitué d'un ester benzylique d'acide hyaluronique ; et
(b) des cellules préadipocytaires ensemencées sur ledit matériau support.

2. Matériel biologique selon la revendication 1, comprenant en outre au moins un membre du groupe consistant en cellules souches mésenchymateuses et cellules endothéliales.

3. Matériel biologique selon la revendication 1 ou 2, dans lequel ledit ester benzylique d'acide hyaluronique est un ester à 100 %, dans lequel tous les groupes carboxyles dudit acide hyaluronique sont estérifiés par un résidu d'alcool benzylique.

4. Matériel biologique selon la revendication 1 ou 2, dans lequel ledit ester benzylique d'acide hyaluronique est un ester à 5 à 99%, dans lequel 5 à 99 % des groupes carboxyles dudit acide hyaluronique sont estérifiés par un résidu alcool benzylique et les autres groupes sont salifiés.

5. Matériel biologique selon l'une quelconque des revendications 1-4, dans lequel ledit matériau support est une structure sous forme d'un matériau spongieux ou non tissé.

6. Matériel biologique selon l'une quelconque des revendications 1-5, dans lequel au moins un membre dudit groupe de cellules préadipocytaires, de cellules souches mésenchymateuses et de cellules endothéliales sont des cellules humaines.

7. Matériel biologique selon l'une quelconque des revendications 1-6 pour le traitement de dommage aux tissus mous.

8. Matériel biologique selon l'une quelconque des revendications 1-7 pour le traitement de reconstruction de dommages aux tissus mous.

9. Préparation injectable pour remplir des défauts et des dépressions de tissus mous, constituée de
(a) un dérivé d'acide hyaluronique totalement hydrosoluble ou un dérivé d'acide hyaluronique partiellement hydrosoluble et
(b) des cellules préadipocytaires en suspension dans la préparation.

10. Préparation injectable selon la revendication 9, comprenant en outre au moins un membre du groupe consistant en cellules souches mésenchymateuses et cellules endothéliales.

11. Préparation injectable selon la revendication 9 ou 10, dans laquelle le dérivé d'acide hyaluronique totalement hydrosolubles est un amide d'acide hyaluronique.

12. Préparation injectable selon la revendication 9 ou 10, dans laquelle le dérivé d'acide hyaluronique totalement hydrosoluble est un dodécylamide d'acide hyaluronique.

13. Préparation injectable selon la revendication 9 ou 10, dans laquelle le dérivé d'acide hyaluronique partiellement hydrosoluble est un ester à 5 à 99 %, dans lequel 5 à 99 % des groupes carboxyles dudit acide hyaluronique sont estérifiés par un résidu alcool benzylique et les autres groupes sont salifiés.

14. Préparation injectable selon la revendication 9 ou 10, dans laquelle le dérivé d'acide hyaluronique partiellement hydrosoluble est un ester à 85 %, dans lequel 85 % des groupes carboxyles dudit acide hyaluronique sont estérifiés par un résidu alcool benzylique et les autres groupes sont salifiés.

15. Préparation injectable selon la revendication 9 ou 10 dans laquelle au moins un membre dudit groupe de cellules préadipocytaires, cellules souches mésenchymateuses et cellules endothéliales sont des cellules humaines.

16. Préparation selon l'une quelconque des revendications 9-15 pour remplir des défauts et des dépressions de tissus mous.

17. Préparation selon l'une quelconque des revendications 9-16 pour remplir des défauts, des dépressions, des rides et des difformités de tissus mous chez un patient le nécessitant.

18. Matériel biologique selon l'une quelconque des revendications 1-5 ou préparation injectable selon l'une quelconque des revendications 9-15 pour une utilisation dans le traitement de reconstruction de dommage aux tissus mous.
